# EUROPEAN PATENT APPLICATION

(11) **EP 4 763 264 A1**
(43) Date of publication of application: **24.06.2026**
(21) Application number: 25158999.0
(22) Date of filing: 20.02.2025
(51) Int. Cl.: A61M 37/00

(54) **HOLLOW MICRONEEDLE**

(30) Priority: 18.12.2024 CN 202411865494
(71) Applicant: Shaanxi Yungu Zhonghui Biopharmaceutical Co., Ltd., Xi'an City, Shaanxi Province 710000 (CN)
(72) Inventor: LI, Ding, Xi'an City, Shaanxi 710000 (CN); YANG, Hao, Xi'an City, Shaanxi 710000 (CN); HU, Chaoyan, Xi'an City, Shaanxi 710000 (CN); DENG, Wu, Xi'an City, Shaanxi 710000 (CN)
(74) Representative: Plougmann Vingtoft a/s

(57) **Abstract**

A hollow microneedle is provided, and relates to the technical field of medical instruments. The hollow microneedle includes a base (1) and a needle head (5) arranged at an upper part (2) of the base. The needle head includes a liquid outlet bevel (9), a rear side surface (10) and two side connecting structures (11). Each of both sides of the liquid outlet bevel is connected with the rear side surface through one side connecting structure. The liquid outlet bevel, the rear side surface and the ends, away from the base, of the two side connecting structures form a needle tip (6), or the rear side surface and the ends, away from the base, of the two side connecting structures form a needle tip. A liquid outlet (8) is formed in the liquid outlet bevel. A liquid outlet channel (7) is formed in the needle head, and a liquid inlet channel is formed in the base.

## Description

### TECHNICAL FIELD

The present disclosure relates to the technical field of medical instruments, in particular to a hollow microneedle.

### BACKGROUND

The microneedle can destroy the surface layer of the skin to perform skin microinjection for drug administration. Through generating a micron-level channel, the microneedle guides directly drugs or active ingredients to the epidermis to participate in microcirculation. The microneedle can penetrate the stratum corneum to form a drug delivery channel without stimulating subcutaneous pain nerves. The microneedle drug administration has broad application prospects. At present, the microneedles on the market mainly include solid microneedles, hollow microneedles, soluble microneedles, coating microneedles and hydrogel microneedles.

The hollow microneedle is of a hollow structure, which can not only carry drugs, but also adjust the speed of releasing drugs or active substances to achieve precise drug administration. However, the existing hollow microneedle is of a conventional tip opening structure, and the needle hole is easily blocked after the skin is punctured. Because of the hollow structure of the needle tip part, the wall thickness of the needle tip section is small, and the mechanical strength is insufficient, easy to occur a situation that the needle is broken or cannot be penetrated into the skin, thus having safety and use hazards.

### SUMMARY

In order to solve the above technical problems, the present disclosure provides a hollow microneedle. The hollow microneedle prevents skin fragments from blocking the liquid outlet in the process of penetrating the skin, so that the stability of the liquid outlet and the integral stability of the needle head are ensured, and the situation that the needle is broken or cannot penetrate the skin is avoided.

In order to achieve the above-mentioned purpose, the present disclosure provides the following solutions.

The present disclosure provides a hollow microneedle. The hollow microneedle includes a base and a needle head arranged at an upper part of the base. The needle head includes a liquid outlet bevel, a rear side surface and two side connecting structures. Each of two sides of the liquid outlet bevel is connected with the rear side surface through one of the side connecting structures. The liquid outlet bevel, the rear side surface and the ends, away from the base, of the two side connecting structures form a needle tip, or the rear side surface and the ends, away from the base, of the two side connecting structures form a needle tip. A liquid outlet is formed in the liquid outlet bevel. A liquid outlet channel is formed in the needle head. A liquid inlet channel is formed in the base. Two ends of the liquid outlet channel are respectively connected with the liquid outlet and one end of the liquid inlet channel. An other end of the liquid inlet channel passes through the bottom of the base.

In some embodiments, the base includes a base station and a housing arranged at an upper part of the base station. The needle head is arranged at an upper part of the housing. The liquid outlet channel includes a middle channel and a hollow zone which are sequentially connected from top to bottom. An upper end of the middle channel is connected with a lower end of the liquid outlet channel. The hollow zone is arranged on the base station and passes through the bottom of the base station. The hollow zone is configured to install a liquid propulsion device.

In some embodiments, the middle channel and the liquid outlet channel which are connected to each other form a tapered channel, and a cross sectional area of the tapered channel is gradually decreased from one end of the tapered channel away from the needle tip to one end of the tapered channel close to the needle tip.

In some embodiments, the upper part of the housing is provided with a plurality of needle heads. The liquid outlet channel includes a plurality of the middle channels. Upper and lower ends of each of the middle channels are respectively connected with a lower end of one of the liquid outlet channels and the hollow zone.

In some embodiments, the side connecting structure includes a lateral vertical surface and a diagonal plane. The bottoms of the liquid outlet bevel, the rear side surface and the lateral vertical surface are connected with an upper surface of the base. Front and rear sides of the lateral vertical surface are respectively connected with one side of the liquid outlet bevel and one side of the rear side surface. A joint of the lateral vertical surface and the rear side surface is cut to form the diagonal plane. The liquid outlet bevel, the rear side surface and the ends, away from the base, of the two diagonal planes form the needle tip.

In some embodiments, the lateral vertical surface is perpendicular to the upper surface of the base. The rear side surface is perpendicular to the upper surface of the base. The rear side surface is a plane or a cambered surface. A joint of the lateral vertical surface and the liquid outlet bevel, a joint of the lateral vertical surface and the diagonal plane, a joint of the diagonal plane and the rear side surface and a joint of the diagonal plane and the liquid outlet bevel are of an arc corner structure.

In some embodiments, the side connecting structure includes a first side surface and a transition connecting structure. The bottoms of the rear side surface and the first side surface are connected with the upper surface of the base. A front side of each of the first side surfaces is connected with a front side of another first side surface, and a rear side of each of the first side surfaces is connected with one side of the rear side surface. The rear side surface and the ends, away from the base, of two first side surfaces form the needle tip. The liquid outlet bevel is arranged on an intersecting line of the two first side surfaces. An inclination angle of the liquid outlet bevel is consistent with an inclination angle of the intersecting line of the two first side surfaces. Each of two sides of the liquid outlet bevel is connected with one of the first side surfaces through one of the transition connecting structures.

In some embodiments, the transition connecting structure includes a second side surface and a third side surface. A lower part of the second side surface is connected with the first side surface. A front side of the second side surface is connected with a lower part of one side of the liquid outlet bevel. A rear side of the second side surface is connected with a front side of the third side surface. A rear side of the third side surface is connected with the first side surface. An upper part of the third side surface is connected with an upper part of one side of the liquid outlet bevel.

In some embodiments, a joint of the second side surface and the liquid outlet bevel, a joint of the second side surface and the third side surface, a joint of the third side surface and the liquid outlet bevel, a joint of the first side surface and the rear side surface and a joint of the two first side surfaces are of an arc corner structure.

In some embodiments, the rear side surface is perpendicular to the upper surface of the base, and the rear side surface is a plane. The liquid outlet bevel is rhombic or drop-shaped. The liquid outlet is formed in a position of the maximum width of the liquid outlet bevel. A width of the liquid outlet bevel is sequentially decreased upward from a position of the liquid outlet, and a width of the liquid outlet bevel is sequentially decreased downward from the position of the liquid outlet.

Compared with the prior art, the present disclosure has the following technical effects.

The hollow microneedle in the present disclosure includes a base and a needle head arranged at an upper part of the base. The needle head includes a liquid outlet bevel, a rear side surface and two side connecting structures. Each of two sides of the liquid outlet bevel is connected with the rear side surface through one side connecting structure. The liquid outlet bevel, the rear side surface and the ends, away from the base, of the two side connecting structures form a needle tip, or the rear side surface and the ends, away from the base, of the two side connecting structures form a needle tip. A liquid outlet is formed in the liquid outlet bevel. The liquid outlet in the present disclosure is formed on the liquid outlet bevel on one side of the needle head, which can not only avoid skin fragments from blocking the liquid outlet in the process of penetrating the skin to ensure that liquid can flow out smoothly from the liquid outlet, but also can increase the thickness of the material of the liquid outlet without affecting the sharpness of the needle tip, so that the position of the liquid outlet is more stable, the stability of the liquid outlet and the integral stability of the needle head are ensured, the mechanical strength of the needle head is improved, and the situation that the needle is broken or cannot penetrate the skin is avoided.

### BRIEF DESCRIPTION OF THE DRAWINGS

To more clearly illustrate the embodiments of the present disclosure or the technical solution in the prior art, the following briefly introduces the accompanying drawings to be used in the embodiments. Apparently, the accompanying drawings in the following description show merely some embodiments of the present disclosure, and those skilled in the art can still derive other drawings from these accompanying drawings without creative efforts.
FIG. 1 is a first three-dimensional structural diagram of a hollow microneedle when provided with one needle head according to a first embodiment of the present disclosure.
FIG. 2 is a second three-dimensional structural diagram of a hollow microneedle when provided with one needle head according to a first embodiment of the present disclosure.
FIG. 3 is a section view of a hollow microneedle when provided with one needle head according to a first embodiment of the present disclosure.
FIG. 4 is a front view of a needle head in a hollow microneedle according to a first embodiment of the present disclosure.
FIG. 5 is a rear view of a needle head in a hollow microneedle according to a first embodiment of the present disclosure.
FIG. 6 is a side view of a needle head in a hollow microneedle according to a first embodiment of the present disclosure.
FIG. 7 is a top view of a needle head in a hollow microneedle according to a first embodiment of the present disclosure.
FIG. 8 is a structure diagram of a hollow microneedle when provided with two needle heads according to a first embodiment of the present disclosure.
FIG. 9 is a structure diagram of a hollow microneedle when provided with three needle heads according to a first embodiment of the present disclosure.
FIG. 10 is a first three-dimensional structural diagram of a hollow microneedle when provided with one needle head according to a second embodiment of the present disclosure.
FIG. 11 is a second three-dimensional structural diagram of a hollow microneedle when provided with one needle head according to a second embodiment of the present disclosure.
FIG. 12 is a front view of a needle head in a hollow microneedle according to a second embodiment of the present disclosure.
FIG. 13 is a rear view of a needle head in a hollow microneedle according to a second embodiment of the present disclosure.
FIG. 14 is a side view of a needle head in a hollow microneedle according to a second embodiment of the present disclosure.
FIG. 15 is a top view of a needle head in a hollow microneedle according to a second embodiment of the present disclosure.
FIG. 16 is a structure diagram of a hollow microneedle when provided with two needle heads according to a second embodiment of the present disclosure.
FIG. 17 is a structure diagram of a hollow microneedle when provided with three needle heads according to a second embodiment of the present disclosure.
FIG. 18 is a structure diagram of a hollow microneedle when provided with one needle head according to a third embodiment of the present disclosure.
FIG. 19 is a section view of a hollow microneedle when provided with one needle head according to a third embodiment of the present disclosure.
FIG. 20 is a first three-dimensional structural diagram of a needle head in a hollow microneedle according to a third embodiment of the present disclosure.
FIG. 21 is a second three-dimensional structural diagram of a needle head in a hollow microneedle according to a third embodiment of the present disclosure.
FIG. 22 is a structure diagram of a hollow microneedle when provided with two needle heads according to a third embodiment of the present disclosure.
FIG. 23 is a structure diagram of a hollow microneedle when provided with three needle heads according to a third embodiment of the present disclosure.
FIG. 24 is a first three-dimensional structural diagram of a hollow microneedle when provided with one needle head according to a fourth embodiment of the present disclosure.
FIG. 25 is a second three-dimensional structural diagram of a hollow microneedle when provided with one needle head according to a fourth embodiment of the present disclosure.
FIG. 26 is a structure diagram of a hollow microneedle when provided with two needle heads according to a fourth embodiment of the present disclosure.
FIG. 27 is a structure diagram of a hollow microneedle when provided with three needle heads according to a fourth embodiment of the present disclosure.

Reference numerals in accompanying drawings: 1, base station; 2, housing; 3, hollow zone; 4, middle channel; 5, needle head; 6, needle tip; 7, liquid outlet channel; 8, liquid outlet; 9, liquid outlet bevel; 10, rear side surface; 11, lateral vertical surface; 12, diagonal plane; 13, first side surface; 14, second side surface; and 15, third side surface.

### DETAILED DESCRIPTION OF THE EMBODIMENTS

The following clearly and completely describes the technical solution in the embodiments of the present disclosure with reference to the accompanying drawings in the embodiments of the present disclosure. Apparently, the described embodiments are merely a part rather than all of the embodiments of the present disclosure. Based on the embodiments in the present disclosure, all other embodiments acquired by those skilled in the art under the premise of without contributing creative labor fall within the scope protected by the present disclosure.

The present disclosure aims to provide a hollow microneedle. The hollow microneedle prevents skin fragments from blocking the liquid outlet in the process of penetrating the skin, so that the stability of the liquid outlet and the integral stability of the whole needle head are ensured, and the situation that the needle is broken or cannot penetrate the skin is avoided.

To make the foregoing objective, features and advantages of the present disclosure clearer and more comprehensible, the present disclosure is further described in detail below with reference to the accompanying drawings and specific embodiments.

### Embodiment I

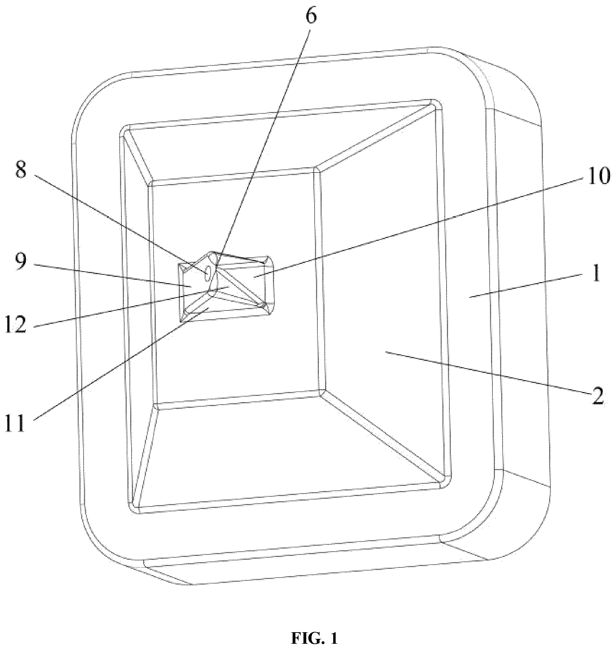

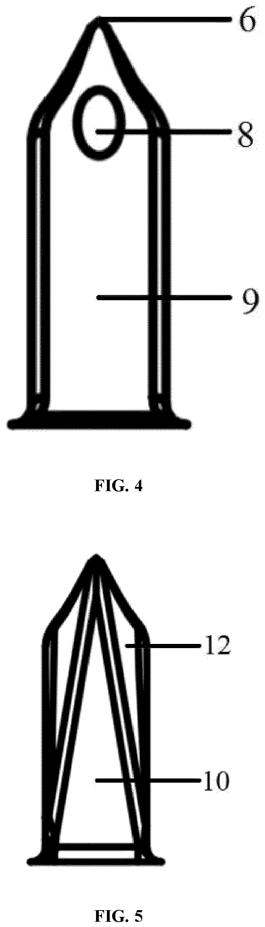

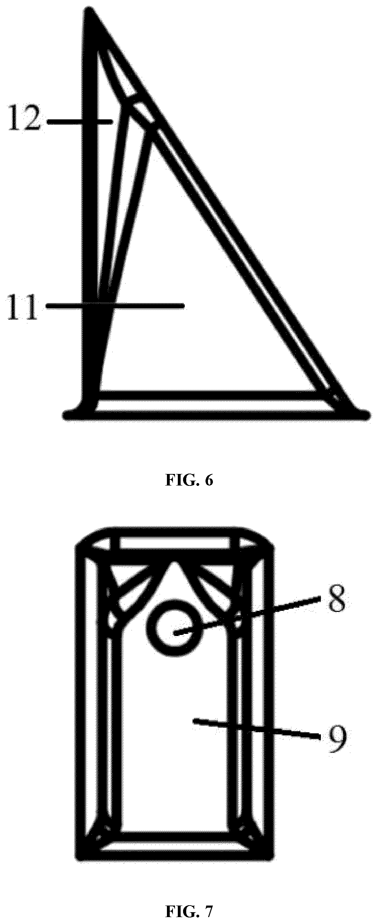

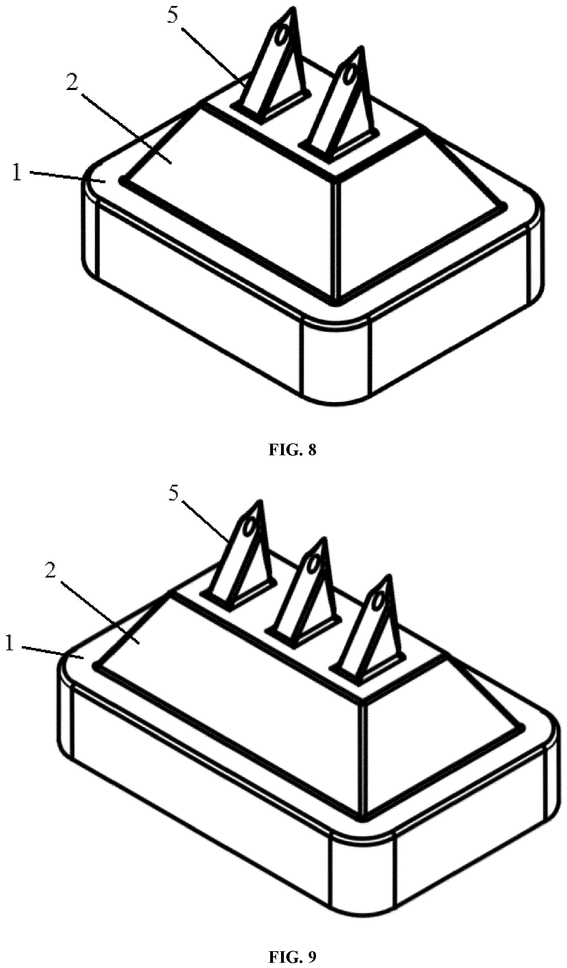

As shown in FIG. 1 to FIG. 9, the embodiment provides a hollow microneedle. The hollow microneedle includes a base and a needle head 5 arranged at an upper part of the base. The needle head 5 and the base are connected along an axial direction of the hollow microneedle. The needle head 5 includes a liquid outlet bevel 9, a rear side surface 10 and two side connecting structures. Each of both sides of the liquid outlet bevel 9 is connected with the rear side surface 10 through one side connecting structure. The bottoms of the side connecting structure and the rear side surface 10 are connected with an upper surface of the base. The rear side surface 10 is vertical to the upper surface of the base, and the rear side surface 10 is a plane or a cambered surface. In the embodiment, the rear side surface 10 located on the other side of the liquid outlet bevel 9 is of a gently structure to avoid from causing cutting damages on the skin. The liquid outlet bevel 9, the rear side surface 10 and the ends, away from the base, of the two side connecting structures form a needle tip 6, or the rear side surface 10 and the ends, away from the base, of the two side connecting structures form a needle tip 6. The formed needle tip 6 facilitates to puncture the skin to enable the needle head 5 to be pricked into the skin. A liquid outlet 8 is formed in the liquid outlet bevel 9. A liquid outlet channel 7 is formed in the needle head 5. A liquid inlet channel is formed in the base. Both ends of the liquid outlet channel 7 are respectively connected with the liquid outlet 8 and one end of the liquid inlet channel, and the other end of the liquid inlet channel passes through the bottom of the base. The liquid inlet channel and the liquid outlet channel 7 form a liquid channel for liquid to flow through. The liquid channel is configured to inject drug liquid or collect tissue fluid.

In the embodiment, the liquid outlet 8 is formed in the liquid outlet bevel 9 on one side of the needle head 5, which can not only avoid skin fragments from blocking the liquid outlet 8 in the process of penetrating the skin to ensure that liquid can flow out smoothly from the liquid outlet 8, but also can increase the thickness of the material of the liquid outlet 8 without affecting the sharpness of the needle tip 6, so that the position of the liquid outlet 8 is more stable, the stability of the liquid outlet 8 and the integral stability of the needle head 5 are ensured, and the mechanical strength of the needle head 5 is improved, thus avoiding the situation that the needle is broken or cannot penetrate the skin. The liquid outlet 8 opened at the side of the needle head 5 can reduce the risk of drug seepage and improve drug administration efficiency.

In some embodiments, specifically, the hollow microneedle can be prepared by using surface projection 3D printing technology and metal powder injection molding technology. In the preparation, the material and the shape of the hollow microneedle can be controlled.

The base includes a base station 1 and a housing 2 arranged at an upper part of the base station 1. The needle head 5 is arranged at an upper part of the housing 2. The liquid outlet channel 7 includes a middle channel 4 and a hollow zone 3 which are sequentially connected from top to bottom. An upper end of the middle channel 4 is connected with a lower end of the liquid outlet channel 7. The hollow zone 3 is arranged on the base station 1 and passes through the bottom of the base station 1. The hollow zone 3 is configured to install a liquid propulsion device.

The base serves as a carrier for the needle head 5, and is also configured to connect the liquid propulsion device, including but not limited to a syringe and the like. The size of the hollow zone 3 of the base can be customized according to the joint part of the liquid propulsion device to ensure the tight combination of the hollow microneedle and the liquid propulsion device.

The base can also play an interception role. When the needle head 5 penetrates the skin, the interception contact between the base and the skin enables the needle head 5 cannot continue to penetrate the deep skin (especially, under the skin) in a condition of penetrating the skin to a specific depth, thus avoiding bleeding and pain.

The middle channel 4 and the liquid outlet channel 7 which are connected to each other form a tapered channel, and the cross sectional area of the tapered channel is gradually decreased from one end away from the needle tip 6 to one end close to the needle tip 6.

In some embodiments, specifically, when 3D printing technology is adopted, the cross sectional area of the channel will be gradually reduced with the printing layer by layer from the bottom. Therefore, the middle channel 4 and the liquid outlet channel 7 which are connected to each other form a tapered channel with a wide lower part and a narrow upper part. When laser drilling is continued after preparation by using metal powder injection molding technology, the hole formed by ablating the material at an entrance port is also tapered, that is, the middle channel 4 and the liquid outlet channel 7 which are connected to each other from a tapered channel with a wide lower part and a narrow upper part.

In the embodiment, the tapered channel is a conical channel, and an included angle between a generatrix of the conical channel and a central axis of the conical channel is 2° to 10°.

The upper part of the housing 2 is provided with multiple needle heads 5. The liquid outlet channel 7 includes multiple middle channels 4. Upper and lower ends of each middle channel 4 are connected with a lower end of one liquid outlet channel 7 and the hollow zone 3, respectively. That is, the middle channels 4 are in one-to-one correspondence with the liquid outlet channel 7, and each middle channel 4 forms a tapered channel with one corresponding liquid outlet channel 7.

In the embodiment, multiple needle heads 5 are arranged in an ordered manner, either in a row or in a regular array. When the needle penetrates the skin, the skin will be depressed due to the surface tension of the skin, so the center distance between adjacent needle heads 5 should be larger than the skin depression size. Otherwise, it is difficult for the needle heads 5 to penetrate the skin. Correspondingly, in the embodiment, the center distance between adjacent needle heads 5 is larger than 200 µm.

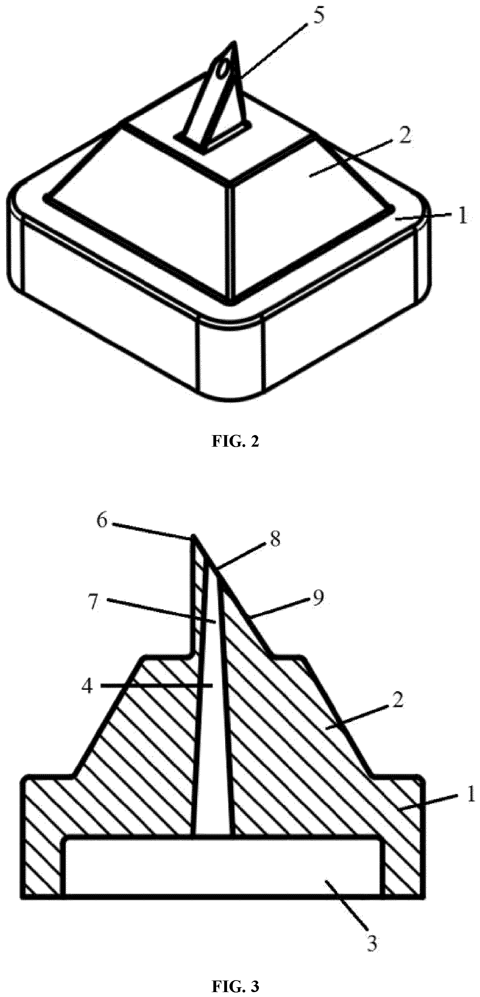

As shown in FIG. 2, FIG. 8 and FIG. 9, the number of needle heads 5 of the hollow microneedle in the embodiment may be one, two or three. It should be noted that the number of needle heads 5 is not limited to the above numbers.

In some embodiments, specifically, the height of the needle head 5 is 610 µm to 850 µm, the width of the bottom of the needle head 5 is 140 µm to 500 µm, and the length of the bottom of a needle body is 140 µm to 600 µm. The diameter of a liquid inlet end of the tapered channel is larger than the diameter of a liquid outlet end of the tapered channel. The diameter of the liquid inlet end (an end away from the needle tip 6) of the tapered channel is 60 µm to 300 µm. The diameter of the liquid outlet end (an end close to the needle tip 6) of the tapered channel is 40 µm to 100 µm.

In some embodiments, specifically, the height of the base is not limited and can be adjusted according to the use needs. In an embodiment, the height of the base station 1 is greater than or equal to 0 µm. When the height of the base tation 1 is 0 µm, that is, when the base station 1 is not provided, the hollow zone 3 is arranged at a lower part of the housing 2.

The housing 2 has a ladder-platform shape that is narrow at the top and wide at the bottom. The width of an upper surface of the housing 2 is 1000 µm. The size of the upper surface of the housing 2 can be set according to the number of the needle heads 5. The length of the upper surface of the housing 2 is at least larger than the length of the bottom of the needle head 5, and the width of the upper surface of the housing 2 is at least larger than the width of the bottom of the needle head 5. The length of the base station 1 is 2200 µm, the width of the base station 1 is 2000 µm, and the height of the base station 1 is 600 µm. The height of the hollow zone 3 can be adjusted according to the length of the joint part of the liquid propulsion device. The height of the hollow zone 3 needs to be greater than 300 µm to ensure the stability of the structure of the base.

When the needle head 5 penetrates the skin, the acting force exerted by the skin on the needle head 5 is sequentially intercepted from the needle tip 6, the liquid outlet bevel 9 and the base, and the needle head 5 does not continue to penetrate the skin. The structural design of the needle head 5 increases the mechanical strength and reduces the risk of needle breakage. The design of the base limits the penetration depth of the needle head 5 to protect the skin.

In some embodiments, specifically, the side connecting structure includes a lateral vertical surface 11 and a diagonal plane 12. The bottoms of the liquid outlet bevel 9, the rear side surface 10 and the lateral vertical surface 11 are connected with the upper surface of the base. Front and rear sides of the lateral vertical surface 11 are connected with one side of the liquid outlet bevel 9 and one side of the rear side surface 10, respectively. The joint of the lateral vertical surface 11 and the rear side surface 10 is cut to form the diagonal plane 12. The liquid outlet bevel 9, the rear side surface 10 and the ends, away from the base, of the two diagonal planes 12 form the needle tip 6.

The existence of the liquid outlet bevel 9 makes the sharpness of the needle tip 6 decrease. Therefore, in the embodiment, two diagonal planes 12 are added at the needle tip 6 to increase the steepness and sharpness of the needle tip 6, which cannot be affected by the shape change of the needle head 5. The design of the two diagonal planes 12 can ensure that the sharpness of the needle tip 6 is controllable, so that the needle head 5 is easier to puncture the skin and reach the deep structure of the skin. Specifically, through setting the two diagonal planes 12, it is enabled that an included angle range of the needle tip 6 is controlled between 10° and 60°.

In some embodiments, specifically, the lateral vertical surface 11 is perpendicular to the upper surface of the base, and the rear side surface 10 is perpendicular to the upper surface of the base.

In some embodiments, specifically, the joint of the lateral vertical surface 11 and the liquid outlet bevel 9, the joint of the lateral vertical surface 11 and the diagonal plane 12, the joint of the diagonal plane 12 and the rear side surface 10 and the joint of the diagonal plane 12 and the liquid outlet bevel 9 are of an arc corner structure, so that cutting damages are avoided from being caused to the skin around the wound after the skin is punctured, and the damage degree to the skin can be reduced to obtain the minimum wound size.

In the specific embodiment, the rear side surface 10 is a plane, and the lateral vertical surface 11 is perpendicular to the rear side surface 10.

### Embodiment II

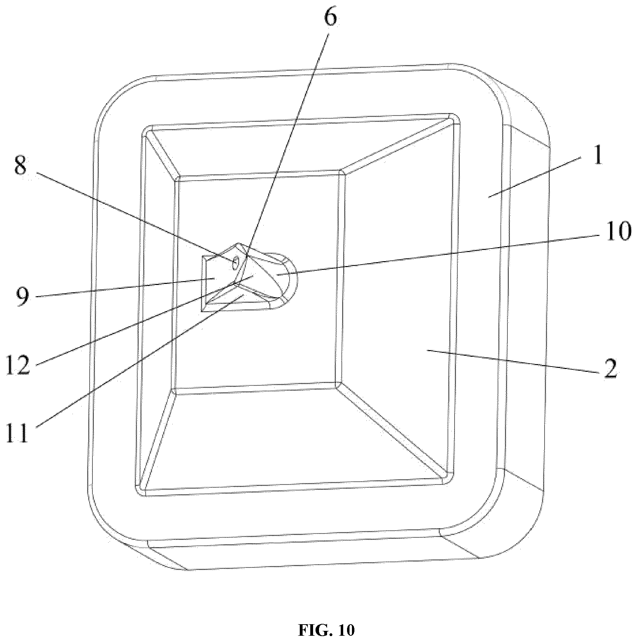

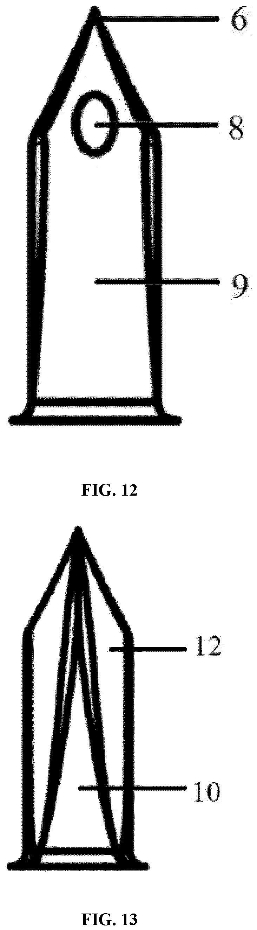

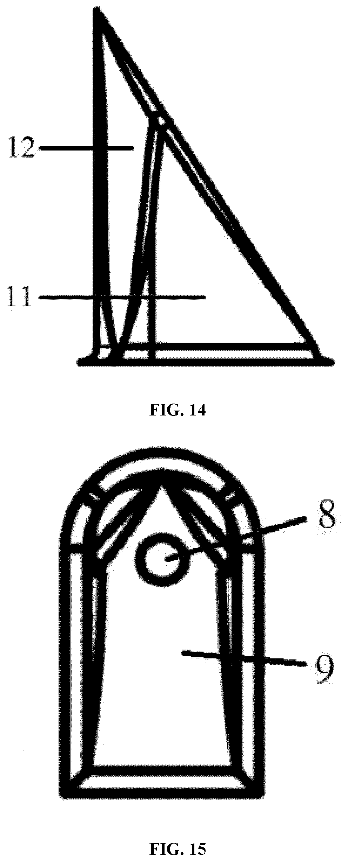

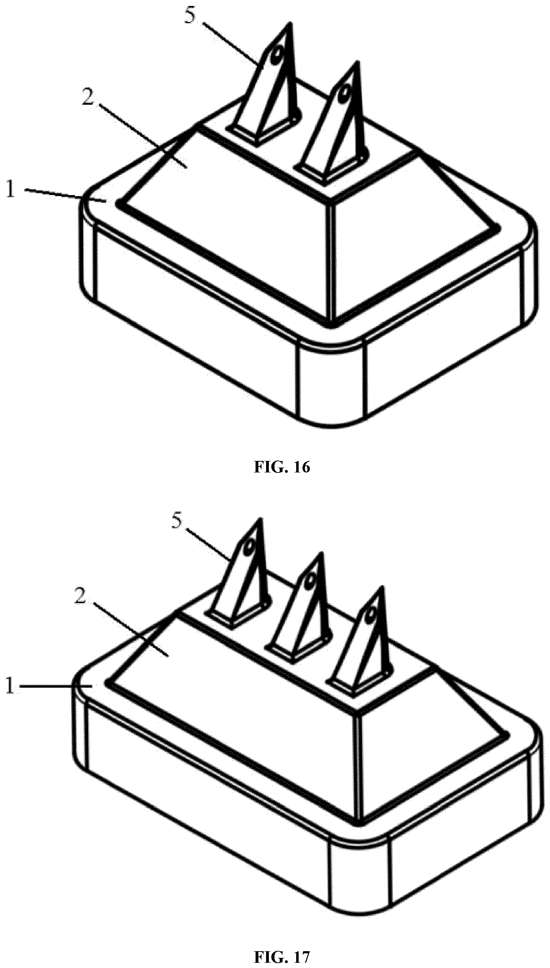

As shown in FIG. 10 to FIG. 17, the embodiment provides a hollow microneedle. The difference between the second embodiment and the first embodiment is the shape of the rear side surface 10. In the specific embodiment, the rear side surface 10 is a cambered surface. Specifically, the rear side surface 10 is a semi-circular arc surface, and the two lateral vertical surfaces 11 are tangent to both ends of the semi-circular arc surface, respectively.

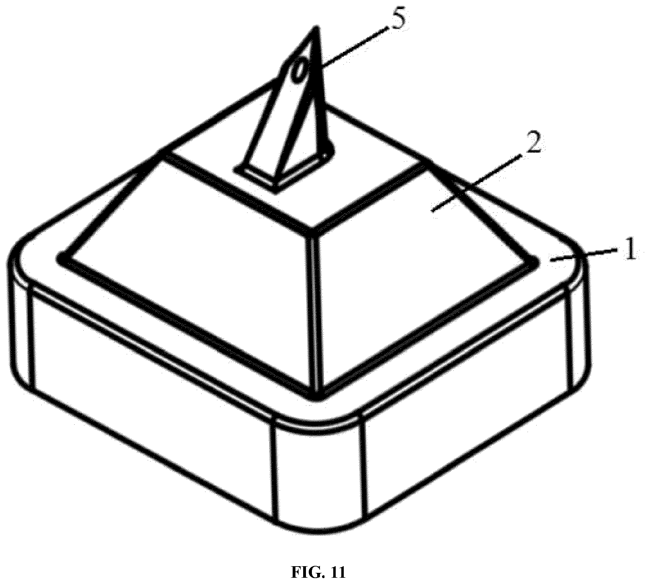

As shown in FIG. 11, FIG. 16 and FIG. 17, the number of needle heads 5 of the hollow microneedle in the embodiment may be one, two or three. It should be noted that the number of needle heads 5 is not limited to the above numbers.

### Embodiment III

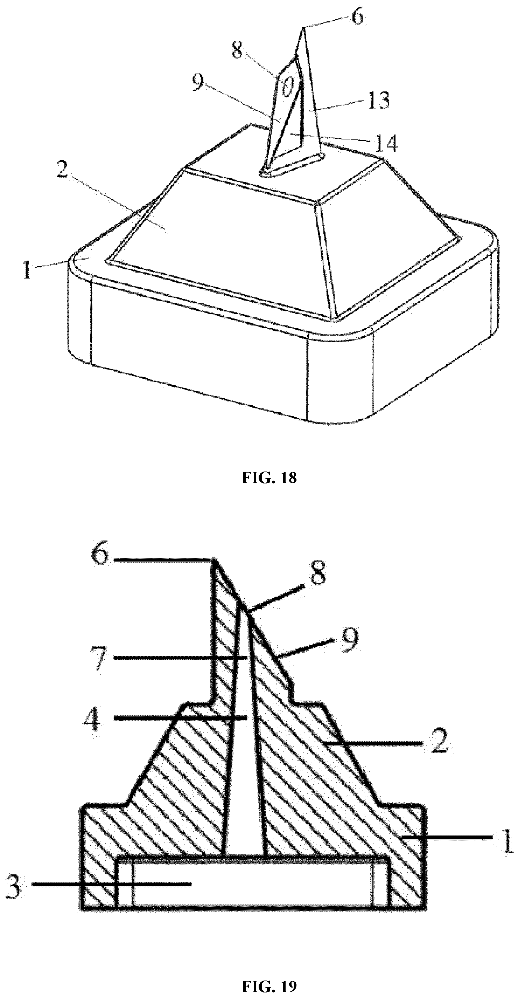

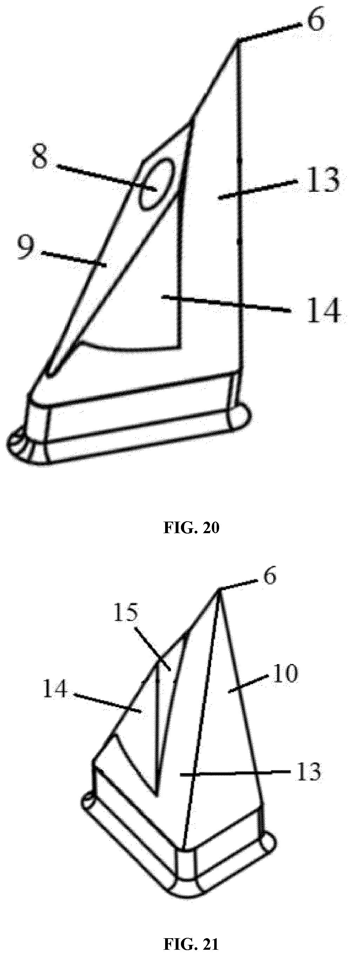

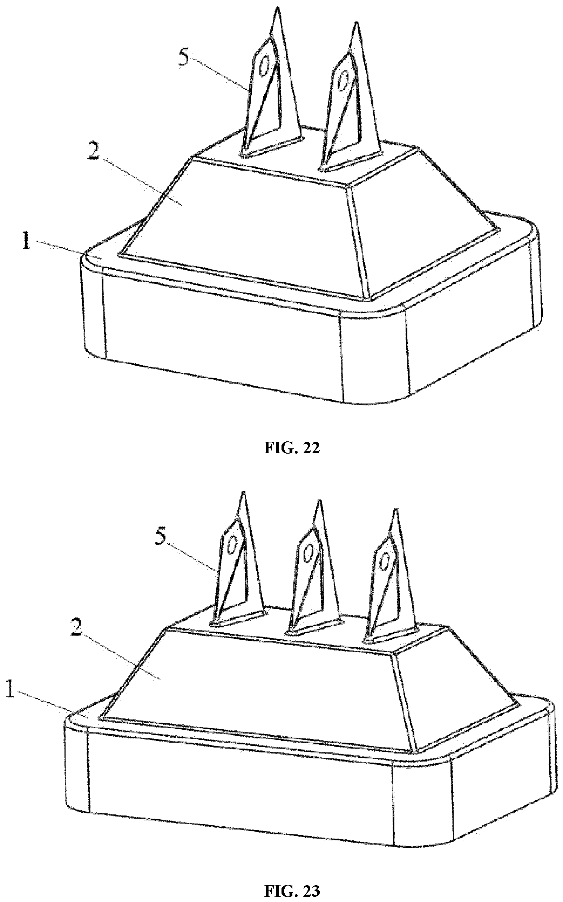

As shown in FIG. 18 to FIG. 23, the embodiment provides a hollow microneedle. The difference between the third embodiment and the first embodiment is a specific structure of the side connecting structure.

In some embodiments, specifically, the side connecting structure includes first side surfaces 13 and a transition connecting structure. The bottoms of the rear side surface 10 and the first side surface 13 are connected with the upper surface of the base. A front side of each first side surface 13 is connected with a front side of another first side surface 13, and a rear side of each first side surface 13 is connected with a side of the rear side surface 10. The rear side surface 10 and the ends, away from the base, of the two first side surfaces 13 form the needle tip 6. The needle tip 6 in the embodiment is a triangular pyramid-shaped tip, and is sharp, and is firm and stable in structure. The liquid outlet bevel 9 is arranged on an intersecting line of the two first side surfaces 13. An inclination angle of the liquid outlet bevel 9 is consistent with an inclination angle of the intersecting line of the two first side surfaces 13. Each of both sides of the liquid outlet bevel 9 is connected with one first side surface 13 through one transition connecting structure. The liquid outlet 8 is of a certain thickness to make the liquid outlet 8 in a complete shape and not be out of shape easily. And, the liquid outlet 8 located in the side of the needle head 5 is not easy to be blocked in the process of puncturing the skin. This design can not only ensure the sharpness of the needle tip 6 to facilitate the piercing of the skin, but also increase the structural stability of the needle head 5.

The transition connecting structure includes a second side surface 14 and a third side surface 15. A lower part of the second side surface 14 is connected with the first side surface 13. A front side of the second side surface 14 is connected with a lower part of one side of the liquid outlet bevel 9. A rear side of the second side surface 14 is connected with a front side of the third side surface 15. A rear side of the third side surface 15 is connected with the first side surface 13. An upper part of the third side surface 15 is connected with an upper part of one side of the liquid outlet bevel 9.

In some embodiments, specifically, the joint of the second side surface 14 and the liquid outlet bevel 9, the joint of the second side surface 14 and the third side surface 15, the joint of the third side surface 15 and the liquid outlet bevel 9, the joint of the first side surface 13 and the rear side surface 10 and the joint of the two first side surfaces 13 are of an arc corner structure, so that cutting damages are avoided from being caused to the skin around the wound after the skin is punctured, and the damage degree to the skin can be reduced to obtain the minimum wound size.

In some embodiments, specifically, the rear side surface 10 is perpendicular to the upper surface of the base, and the rear side surface 10 is a plane.

In the specific embodiment, the liquid outlet bevel 9 is rhombic, and an included angle of two side edges of an upper part of the rhombic liquid outlet bevel 9 is larger than an included angle of two side edges of a lower part of the rhombic liquid outlet bevel 9. The liquid outlet 8 is formed in the position of the maximum width of the liquid outlet bevel 9. The width of the liquid outlet bevel 9 is sequentially decreased upward from the position of the liquid outlet 8. The width of the liquid outlet bevel 9 is sequentially decreased downward from the position of the liquid outlet 8. This design can not only ensure the thickness of the liquid outlet 8, but also ensure that the sharpness of the needle head 5 is not greatly affected.

As shown in FIG. 18, FIG. 22 and FIG. 23, the number of needle heads 5 of the hollow microneedle in the embodiment may be one, two or three. It should be noted that the number of needle heads 5 is not limited to the above numbers.

### Embodiment IV

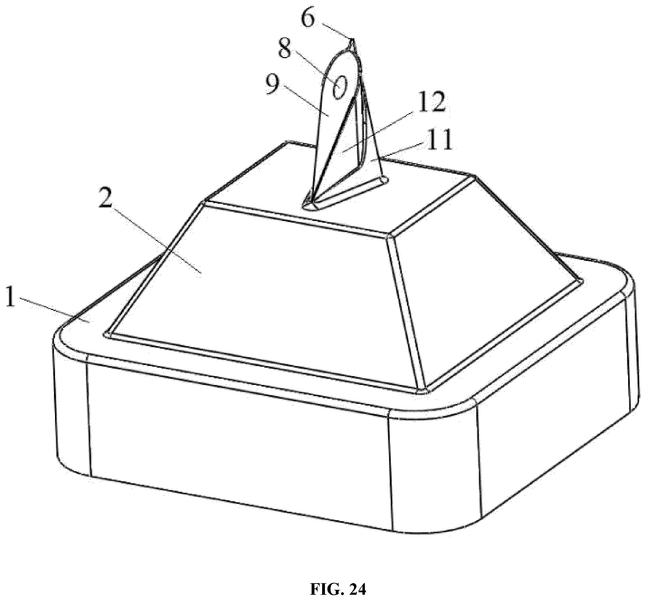

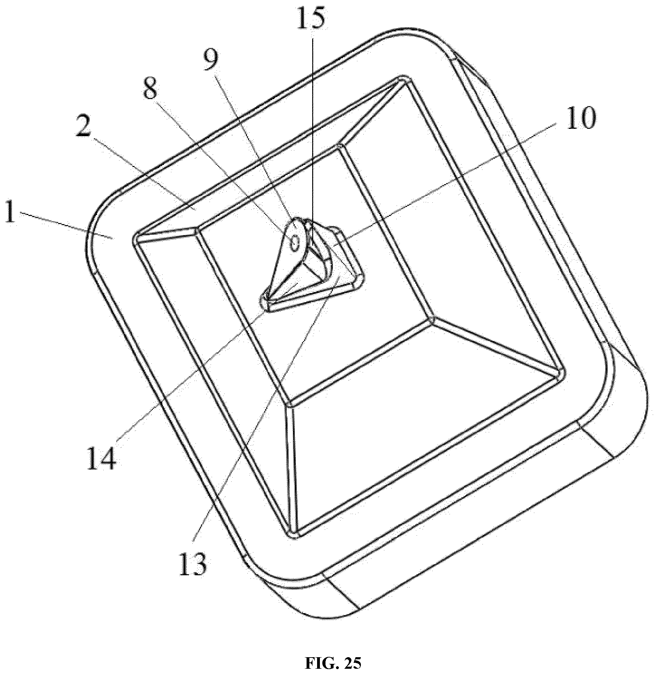

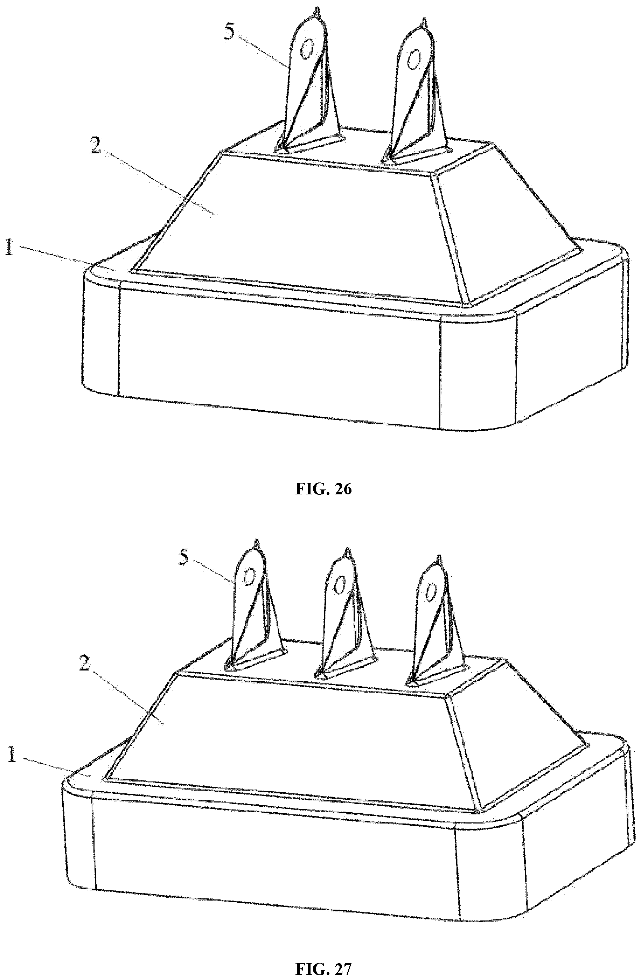

As shown in FIG. 24 to FIG. 27, the embodiment provides a hollow microneedle. The difference between the fourth embodiment and the third embodiment is the shape of the liquid outlet bevel 9. In the specific embodiment, the liquid outlet bevel 9 is drop-shaped, and the tip of the drop-shaped liquid outlet bevel 9 is located at the lower part. The liquid outlet 8 is formed in the position of the maximum width of the liquid outlet bevel 9. The width of the liquid outlet bevel 9 is sequentially decreased upward from the position of the liquid outlet 8. The width of the liquid outlet bevel 9 is sequentially decreased downward from the position of the liquid outlet 8. This design can not only ensure the thickness of the liquid outlet 8, but also ensure that the sharpness of the needle head 5 is not greatly affected.

As shown in FIG. 24, FIG. 26 and FIG. 27, the number of needle heads 5 of the hollow microneedle in the embodiment may be one, two or three. It should be noted that the number of needle heads 5 is not limited to the above numbers.

Specific examples are used in the specification for illustration of the principles and implementations of the present disclosure. The description of the above-mentioned embodiments is merely used to help understand the method and the core principles of the present disclosure; and meanwhile, those skilled in the art can make various modifications in terms of specific embodiments and application scope in accordance with the teachings of the present disclosure. In summary, the contents of this specification should not be understood as the limitation of the present disclosure.

## Claims

1. A hollow microneedle, comprising a base and a needle head arranged at an upper part of the base, wherein the needle head comprises a liquid outlet bevel, a rear side surface and two side connecting structures; each of two sides of the liquid outlet bevel is connected with the rear side surface through one of the side connecting structures; and the liquid outlet bevel, the rear side surface and the ends, away from the base, of the two side connecting structures form a needle tip, or the rear side surface and the ends, away from the base, of the two side connecting structures form a needle tip; and wherein a liquid outlet is formed in the liquid outlet bevel, a liquid outlet channel is formed in the needle head, a liquid inlet channel is formed in the base, two ends of the liquid outlet channel are respectively connected with the liquid outlet and one end of the liquid inlet channel, and an other end of the liquid inlet channel passes through a bottom of the base.

2. The hollow microneedle according to claim 1, wherein the base comprises a base station and a housing arranged at an upper part of the base station, the needle head is arranged at an upper part of the housing; the liquid outlet channel comprises a middle channel and a hollow zone which are sequentially connected from top to bottom, an upper end of the middle channel is connected with a lower end of the liquid outlet channel, the hollow zone is arranged on the base station and passes through the bottom of the base station, and the hollow zone is configured to install a liquid propulsion device.

3. The hollow microneedle according to claim 2, wherein the middle channel and the liquid outlet channel which are connected to each other form a tapered channel, a cross sectional area of the tapered channel is gradually decreased from one end of the tapered channel away from the needle tip to one end of the tapered channel close to the needle tip.

4. The hollow microneedle according to claim 2, wherein the upper part of the housing is provided with a plurality of needle heads, the liquid outlet channel comprises a plurality of the middle channels, and upper and lower ends of each of the middle channels are respectively connected with a lower end of one of the liquid outlet channels and the hollow zone.

5. The hollow microneedle according to claim 1, wherein the side connecting structure comprises a lateral vertical surface and a diagonal plane; the bottoms of the liquid outlet bevel, the rear side surface and the lateral vertical surface are connected with an upper surface of the base, front and rear sides of the lateral vertical surface are respectively connected with one side of the liquid outlet bevel and one side of the rear side surface; a joint of the lateral vertical surface and the rear side surface is cut to form the diagonal plane; and the liquid outlet bevel, the rear side surface and the ends, away from the base, of the two diagonal planes form the needle tip.

6. The hollow microneedle according to claim 5, wherein the lateral vertical surface is perpendicular to the upper surface of the base, the rear side surface is perpendicular to the upper surface of the base, the rear side surface is a plane or a cambered surface; and a joint of the lateral vertical surface and the liquid outlet bevel, a joint of the lateral vertical surface and the diagonal plane, a joint of the diagonal plane and the rear side surface and a joint of the diagonal plane and the liquid outlet bevel are of an arc corner structure.

7. The hollow microneedle according to claim 1, wherein the side connecting structure comprises a first side surface and a transition connecting structure; the bottoms of the rear side surface and the first side surface are connected with the upper surface of the base, a front side of each of the first side surfaces is connected with a front side of another first side surface, a rear side of each of the first side surfaces is connected with one side of the rear side surface; the rear side surface and the ends, away from the base, of two first side surfaces form the needle tip, the liquid outlet bevel is arranged on an intersecting line of the two first side surfaces, an inclination angle of the liquid outlet bevel is consistent with an inclination angle of the intersecting line of the two first side surfaces, and each of two sides of the liquid outlet bevel is connected with one of the first side surfaces through one of the transition connecting structures.

8. The hollow microneedle according to claim 7, wherein the transition connecting structure comprises a second side surface and a third side surface, a lower part of the second side surface is connected with the first side surface, a front side of the second side surface is connected with a lower part of one side of the liquid outlet bevel, a rear side of the second side surface is connected with a front side of the third side surface, a rear side of the third side surface is connected with the first side surface, and an upper part of the third side surface is connected with an upper part of one side of the liquid outlet bevel.

9. The hollow microneedle according to claim 8, wherein a joint of the second side surface and the liquid outlet bevel, a joint of the second side surface and the third side surface, a joint of the third side surface and the liquid outlet bevel, a joint of the first side surface and the rear side surface and a joint of the two first side surfaces are of an arc corner structure.

10. The hollow microneedle according to claim 7, wherein the rear side surface is perpendicular to the upper surface of the base, the rear side surface is a plane; the liquid outlet bevel is rhombic or drop-shaped, the liquid outlet is formed in a position of the maximum width of the liquid outlet bevel, a width of the liquid outlet bevel is sequentially decreased upward from a position of the liquid outlet, and a width of the liquid outlet bevel is sequentially decreased downward from the position of the liquid outlet.
